# EUROPÄISCHE PATENTSCHRIFT

(11) **EP 0 815 070 B1**
(45) Veröffentlichungstag und Bekanntmachung des Hinweises auf die Patenterteilung: **14.06.2000**
(21) Anmeldenummer: 96908048.0
(22) Anmeldetag: 15.03.1996
(51) Int. Cl.: C07C 67/36, C07C 69/33

(54) **VERFAHREN ZUR HERSTELLUNG VON 3-PENTENSÄUREESTERN DURCH CARBONYLIERUNG VON ALKOXYBUTENEN**
METHOD OF PRODUCING 3-PENTENIC ACID ESTERS BY CARBONYLATION OF ALKOXYBUTENES
PROCEDE DE FABRICATION DE 3-ESTERS D'ACIDE PENTENIQUE PAR CARBONYLATION D'ALCOXYBUTENES

(30) Priorität: 22.03.1995 DE 19510324
(43) Veröffentlichungstag der Anmeldung: 07.01.1998
(73) Patentinhaber: BASF AKTIENGESELLSCHAFT, 67056 Ludwigshafen (DE)
(72) Erfinder: LANGGUTH, Ernst, D-67281 Kirchheim (DE); SCHNEIDER, Regina, D-67136 Fussgönheim (DE); LIPPERT, Ferdinand, D-67098 Bad Dürkheim (DE); HÖHN, Arthur, D-67281 Kirchheim (DE)
(86) Internationale Anmeldenummer: EP9601123
(87) Internationale Veröffentlichungsnummer: WO9629300

(56) Entgegenhaltungen:
- EP-A- 0 217 407
- EP-A- 0 478 471
- EP-A- 0 514 288
- US-A- 5 166 421
- US-A- 5 495 041

## Beschreibung

Die vorliegende Erfindung betrifft ein Verfahren zur Herstellung von 3-Pentensäureestern durch Carbonylierung von Alkoxybutenen in Gegenwart eines Katalysators und eines Lösungsmittels bei erhöhter Temperatur und erhöhtem Druck.

Aus EP-A 301 450 und EP-A 351 616 sind Verfahren zur Herstellung von Pentensäurealkylestern durch Umsetzen von Butadien mit Kohlenstoffmonoxid und Alkoholen in Gegenwart von Cobaltcarbonylkomplexen und tertiären Stickstoffbasen bekannt. Dabei werden hohe Drücke von 120 bis 700 bar benötigt und es bilden sich Mischungen aus 2-, 3- und 4-Pentensäureestern.

GB-A 1 110 405 beschreibt ein Verfahren zur Herstellung von Pentenestern durch Carbonylierung von Butadien in Gegenwart eines Alkohols mit Platin-, Palladium- und/oder Nickelkatalysatoren. Auch hier sind hohe Drücke von 100 bis 1000 bar erforderlich.

Aus EP-A 60 734 ist zwar ein Verfahren zur Herstellung von Pentenestern durch Carbonylierung von Butadien in Gegenwart eines Alkohols, eines Halogenwasserstoffs und eines Palladium-Katalysators bei niedrigeren Drücken um 150 bar bekannt. Aus US-A-5,166,421 ist ein Verfahren zur Herstellung von Pentensäureestern aus Alkoxybutenen an einem Rhodium enthaltenden Katalysator bekannt. Nachteilig bei diesen Verfahren ist der erforderliche hohe Überschuß an korrosiv wirkendem Halogenwasserstoff (Molverhältnis Halogenwasserstoff zu Palladium von 20 bis 100 zu 1).

Nach EP-A 284 170 und EP-A 271 145 können Pentenester durch Carbonylierung von Butadien in Gegenwart von Alkoholen mit Palladium-Verbindungen, Phosphinen und Säuren hergestellt werden. Dabei entsteht der 3-Pentenester nicht in reiner Form, sondern im Gemisch mit seinen isomeren Verbindungen.

Ein anderer Weg zur Herstellung von β,γ-ungesättigten Estern wird in US 4 622 416 beschrieben. Durch Carbonylierung von Allylethern werden unter Katalyse von Nickel-, Cobalt- oder Eisenhalogeniden die Ester erhalten. Nachteilig bei diesem Verfahren ist die Bildung von Produktgemischen. Bei der Carbonylierung von 8-Methoxy-1,6-octadien entstehen neben 3,8-Nonadiensäuremethylestern noch drei cyclische Carbonsäureverbindungen. Zufriedenstellende Selektivitäten (maximal 91 %) können erst bei einem Druck ab 170 bar und einer Temperatur von 150°C erreicht werden. Unter diesen Bedingungen ist der Katalysatorverlust durch Bildung von flüchtigen Nickel-Verbindungen sehr groß.

Die EP-A 217 407 beschreibt die Carbonylierung von Allylethern unter PdCl₂/CuCl₂-Katalyse zu ungesättigten Estern. Dabei werden der Reaktionsmischung zur extraktiven Abtrennung des Produktes große Mengen Tetrabutylammoniumchlorid (25 mol-% bezüglich Edukt) zugesetzt. Dieser Zusatz führt zu einer starken Abscheidung von metallischem Palladium.

Aus EP-A 514 288, EP-A 478 471 und EP-A 433 191 ist die doppelte Carbonylierung von 1,4-Butendiolen und 1,4-Dialkoxybutenen zu Dehydroadipinsäure(diestern) mit Palladium-Verbindungen und Chloriden wie Alkalimetall-, Erdalkalimetall- oder quartären Ammonium- oder Phosphoniumhalogeniden bekannt. Diese Verfahren erfordern einen großen Chlorid-Überschuß (typische Molverhältnisse Pd zu Chlorid von 1:17 bis 1:27) oder aber große PdCl2-Mengen von etwa 20 mol-%, bezogen auf das Edukt.

Aufgabe der vorliegenden Erfindung war daher die Bereitstellung eines Verfahrens zur Herstellung von 3-Pentensäureestern mit einem möglichst geringen Anteil an isomeren 2- und 4-Pentensäureestern durch Carbonylierung von Alkoxybutenen in Gegenwart eines Katalysators auf Basis von Palladium unter milden Bedingungen.

Demgemäß wurde ein Verfahren zur Herstellung von 3-Pentensäureestern durch Carbonylierung von Alkoxybutenen in Gegenwart eines Katalysators und eines Lösungsmittels bei erhöhter Temperatur und erhöhtem Druck, gefunden, indem man mindestens ein C₁-C₁₀-Alkoxybuten, in dem die Alkoxygruppe in Allylstellung zur Doppelbindung steht, mit Kohlenstoffmonoxid bei einer Temperatur im Bereich von 60 bis 140°C und einem Kohlenstoffmonoxid-Partialdruck im Bereich von 3 bis 30 MPa in Gegenwart eines Katalysators auf der Basis von Palladium in Abwesenheit Halogenid-haltiger Zusatzstoffe oder in Gegenwart eines Zusatzstoffes ausgewählt aus der Gruppe bestehend aus Chloriden, Säuren, Stickstoff-haltige Liganden und Phosphor-haltige Liganden bei einem molaren Verhältnis von Zusatzstoff zu Katalysator von 0,1 bis 10 umsetzt.

Des weiteren wurde ein homogenes Katalysatorsystem gefunden, bei dem keine Katalysatordesaktivierung durch Palladiumabscheidung auftritt, wenn man die Carbonylierung zusätzlich in Gegenwart von quartären Ammonium- oder Phosphoniumsalzen oder speziellen Phosphinen durchführt.

Als Ausgangsstoffe im erfindungsgemäßen Verfahren setzt man mindestens ein C₁-C₁₀-Alkoxybuten, vorzugsweise ein C₁-C₄-Alkoxybuten, in dem die Alkoxygruppe in Allylstellung zur Doppelbindung steht, ein. Bevorzugt seien genannt 3-Methoxy-1-buten, 3-Ethoxy-1-buten, 3-n-Propoxy-1-buten, 3-n-Butoxy-1-buten, trans-1-Methoxy-2-buten, trans-1-Ethoxy-2-buten, trans-1-n-Propoxy-2-buten, trans-1-n-Butoxy-2-buten, cis-1-Methoxy-2-buten, cis-1-Ethoxy-2-buten, cis-1-n-Propoxy-2-buten, cis-1-n-Butoxy-2-buten, sowie Mischungen davon, insbesondere eine Mischung aus 3-Methoxy-1-buten, trans-1-Methoxy-2-buten und cis-1-Methoxy-2-buten.

Die Ausgangsverbindungen können gemäß US 2,922,822 durch sauer katalysierte Alkoholaddition an Butadien hergestellt werden.

Als Katalysator setzt man erfindungsgemäß einen Katalysator auf der Basis von Palladium ein. Bevorzugt verwendet man Palladium-Verbindungen in den Oxidationsstufen 0, +1 oder +2, die als Palladiumsalze oder Palladiumkomplexe vorliegen können, insbesondere PdCl₂, PdCl₂(Benzonitril)₂, PdCl₂(Acetonitril)₂, Pd(OAc)₂, Bis-allylchloropalladiumkomplexe und Dichlorodiphosphinpalladiumkomplexe. Entsprechende Verbindungen sind dem Fachmann bekannt, beispielsweise aus Dictionary of Organometallic Compounds, Vol.2, 1984, Chapman and Hall, S. 1484-1544.

Üblicherweise liegt das Molverhältnis von Palladiumverbindung zu Alkoxybuten (oder der Summe der Mole der eingesetzten Alkoxybutenen) im Bereich von 0,1:1 bis 10:1, vorzugsweise von 0,5:1 bis 5:1.

Erfindungsgemäß führt man die Reaktion in Abwesenheit Halogenid-haltiger Zusatzstoffe durch oder man erhöht die Aktivität und/ oder die Stabilität der Palladium-Katalysatoren durch Zusatz von Chloriden, Säuren, Stickstoff- oder Phosphor-haltigen Liganden (im folgenden insgesamt als Zusatzstoffe bezeichnet) bei einem molaren Verhältnis von Zusatzstoff zu Katalysator von 0,1 bis 10, vorzugsweise 0,5 bis 4. Als Chloride verwendet man bevorzugt Alkalimetall-, Erdalkalimetall-, Übergangsmetall-, quartäre Ammonium- und Phosphonium- chloride wie

Lithium-, Natrium-, Kaliumchlorid, vorzugsweise Natriumchlorid, Magnesium-, Calcium-, Strontium-, Bariumdichlorid, vorzugsweise Calciumdichlorid, Kupferdichlorid, Silberchlorid, Goldtrichlorid, vorzugsweise Kupferdichlorid, sowie Verbindungen der allgemeinen Formel R¹R²R³R⁴NCl, R¹R²R³R⁴PCl oder (R⁵)₃N=P=N(R⁵)₃, wobei R¹ bis R⁴ gleich oder verschieden sind und für aliphatische Gruppen mit 1 bis 10 Kohlenstoffatomen, bevorzugt 4 bis 8 Kohlenstoffatomen und/oder für gegebenenfalls substituierte Arylgruppen stehen, R⁵ eine Arylgruppe mit 6-10 Kohlenstoffatomen darstellt, die gegebenenfalls substituiert ist mit Alkylgruppen, Alkoxygruppen oder Alkoxycarbonylgruppen mit 1-4 C-Atomen oder mit Halogen, vorzugsweise verwendet man Tetrabutylammoniumchlorid, Tetrabutylphosphoniumchlorid und Bis-(triphenylphosphin)imimiumchlorid.

Bevorzugt setzt man als Säuren anorganische und organische Protonensäuren wie Salzsäure, Schwefelsäure, Phosphorsäure, Tetrafluorborsäure oder Sulfonsäuren wie Methansulfonsäure und p-Toluolsulfonsäure oder Lewis-Säuren wie Bortrifluorid-Diethylether-Komplex und Aluminiumtrichlorid.

Bevorzugt setzt man als Phosphor-Verbindungen Phosphine ein mit der allgemeinen Formel R⁶R⁷R⁸P, wobei R⁶ bis R⁸ gleich oder verschieden sind und für aliphatische Gruppen mit 1 bis 10 Kohlenstoffatomen, bevorzugt 4 bis 8 C-Atome, für gegebenenfalls substituierte Aryl- oder gegebenenfalls substituierte Heteroarylgruppen mit 6 bis 10 C-Atomen, bevorzugt Phenyl-, Pyridyl- und Pyrimidyl-Gruppen stehen. Als Beispiele seien Triphenylphosphin, Tricyclohexylphosphin, Tris-(2-methoxyphenyl)-phosphin, Tris-(3-methoxyphenyl)-phosphin, Tris-(4-methoxyphenyl)-phosphin und 2-Diphenylphosphinopyridin genannt.

Als Phosphor-Verbindungen können auch mehrzähnige Chelatliganden wie Bis-(diphenylphosphino)-methan, 1,2-Bis(diphenylphosphino)-ethan, 1,3-Bis-(diphenylphosphino)-propan, 1,4-Bis-(diphenylphosphino)-butan, 1,2-Bis-(diphenylphosphino)-ethan und Bis-(di-tert.-butylphosphino)-methan eingesetzt werden.

Erfindungsgemäß führt man die Carbonylierung bei einer Temperatur im Bereich von 60 bis 140, vorzugsweise von 80 bis 120°C, und einem Kohlenstoffmonoxid-Partialdruck im Bereich von 3 bis 30, vorzugsweise von 5 bis 15 MPa, durch.

Des weiteren kann man die Carbonylierung diskontinuierlich oder kontinuierlich durchführen.

Ferner kann man die Carbonylierung in Gegenwart eines Lösungsmittels durchführen, wobei man in der Regel das Gewichtsverhältnis von Lösungsmittel zu Alkoxybuten(en) im Bereich von 0,5:1 bis 15:1, vorzugsweise von 2:1 bis 10:1 wählt.

Als Lösungsmittel setzt man ein
- aliphatische, cycloaliphatische oder aromatische Alkohole mit einem bis zehn C-Atomen, bevorzugt von einem bis vier C-Atomen, vorzugsweise setzt man solche Alkohole ROH ein, deren RO-Rest demjenigen des C₁-C₁₀-Alkoxy-Restes des eingesetzten Alkoxybutens entspricht, bevorzugt Methanol, Ethanol, n-Propanol und n-Butanol;
- aliphatische oder aromatische Nitrile mit zwei bis zehn C-Atomen, vorzugsweise Benzonitril, Acetonitril, Propionsäurenitril;
- Harnstoffe mit fünf bis fünfzehn C-Atomen, vorzugsweise Tetramethylharnstoff, Dimethylethylenharnstoff, Dimethyl. propylenharnstoff,
- Säureamide mit drei bis zehn C-Atomen, vorzugsweise Dimethylformamid, Dibutylformamid, Dimethylacetamid, N-Methyl-2-pyrrolidon;
- Carbaminsäureester mit vier bis dreizehn C-Atomen wie 3-Methyl-2-oxazolidinon;
- Kohlenwasserstoffe mit fünf bis zehn C-Atomen wie Benzol und Toluol;
- Ether mit zwei bis sechzehn C-Atomen wie Methyl-tert.butylether, Diphenylether;
sowie Mischungen davon.

Die erfindungsgemäß herstellbaren 3-Pentensäureester sind wichtige Zwischenverbindungen zur Herstellung von beispielsweise Adipinsäure, Caprolactam und Caprolacton sowie deren Polymere und Copolymere wie Polyamid-6 und Polyamid-66.

Die Vorteile des erfindungsgemäßen Verfahrens gegenüber Verfahren aus dem Stand der Technik sind, daß hohe Drücke, d.h. Drücke von mehr als 30 MPa vermieden werden können, daß hohe Ausbeuten erreicht werden, daß 3-Pentensäureester mit hoher Isomerenreinheit erhalten werden, daß man Isomerengemische von Alkoxybutenen einsetzen kann, daß das Verfahren auch kontinuierlich ausgeübt werden kann und daß der Katalysator ohne großen Aktivitätsverlust recyclisiert werden kann.

### Beispiele

Bei allen Beispielen wurden die Ausbeuten gaschromatographisch ermittelt. Dabei konnte kein 4-Pentensäureester nachgewiesen werden. In den Beispielen 1 bis 19 entstanden weniger als 2 % 2-Pentensäureester, bezogen auf den jeweiligen 3-Pentensäureester.

### Beispiel 1

Eine Mischung aus 61,48 mmol 3-Methoxy-1-buten, 48,72 mmol trans-1-Methoxy-2-buten, 5,8 mmol cis-1-Methoxy-2-buten, 5,6 mmol PdCl₂ und 45 g Methanol wurde in einem 300 ml Autoklaven bei Raumtemperatur mit 10 MPa Kohlenstoffmonoxid versetzt. Anschließend wurde auf 80°C aufgeheizt und 5 h bei dieser Temperatur und dem sich eingestellten Eigendruck (12 MPa) gerührt. Danach wurde auf Raumtemperatur abgekühlt und der Druck auf Atmosphärendruck gebracht. Die Ausbeute an 3-Pentensäuremethylester betrug 60%.

### Beispiele 2 bis 4

Eine Mischung aus 53,0 mmol 3-Methoxy-1-buten, 42,0 mmol trans-1-Methoxy-2-buten, 5,0 mmol cis-1-Methoxy-2-buten, 2,5 mmol PdCl₂ und 40 g eines Lösungsmittels (siehe Tabelle 1) wurde in einem 300 ml Autoklaven bei Raumtemperatur mit 5 MPa Kohlenstoffmonoxid versetzt. Anschließend wurde aufgeheizt (Temperatur siehe Tabelle 1) und 5 h bei dieser Temperatur und einem Druck von 10 MPa gerührt. Danach wurde auf Raumtemperatur abgekühlt und der Druck auf Atmosphärendruck gebracht. Die Ausbeute an 3-Pentensäuremethylester ist ebenfalls Tabelle 1 zu entnehmen.

**Tabelle 1**

| Beispiel | Lösungsmittel | Temperatur [°C] | Ausbeute [%] |
|---|---|---|---|
| 2 | Dimethylpropylenharnstoff | 80 | 57 |
| 3 | Benzonitril | 100 | 61 |
| 4 | MeOH/Benzonitril (1:1) | 100 | 61 |

### Beispiele 5 bis 18

Eine Mischung aus 53,0 mmol 3-Methoxy-1-buten, 42,0 mmol trans-1-Methoxy-2-buten, 5,0 mmol cis-1-Methoxy-2-buten, 2,5 mmol PdCl₂, 2,5 bis 10 mmol eines Zusatzstoffes (siehe Tabelle 2) und 40 g eines Lösungsmittels (siehe Tabelle 2) wurde in einem 300 ml Autoklaven bei Raumtemperatur mit 10 MPa Kohlenstoffmonoxid versetzt. Anschließend wurde auf 100°C aufgeheizt und 5 h bei dieser Temperatur und dem sich eingestellten Eigendruck (<13 MPa) gerührt. Danach wurde auf Raumtemperatur abgekühlt und der Druck auf Atmosphärendruck gebracht. Die Ausbeute an 3-Pentensäuremethylester ist ebenfalls Tabelle 2 zu entnehmen.

**Tabelle 2**

| Bsp. | Lösungsmittel | Zusatzstoff (Mol pro Mol Pd) | Ausbeute [%] |
|---|---|---|---|
| 5 | Benzonitril | CuCl₂ (1) | 75 |
| 6 | " | AlCl₃ (1) | 72 |
| 7 | " | Bu₄PCl (2) | 39 |
| 8 | " | Bu₄NCl (1) | 60 |
| 9 | " | Ph₂PPy, MSS (1/1) | 74 |
| 10 | " | Ph₂PPy, MSS (4/4) | 56 |
| 11 | NMP | Bu₄NCl (1) | 71 |
| 12 | " | P(o-CH₃OC₆H₄)₃ (1) | 77 |
| 13 | " | Ph₂PPy, p-TosOH (4/4) | 63 |
| 14 | 3-Methyl-2-oxazolidinon | Ph₂PPy, MSS (4/4) | 58 |
| 15 | " | Bu₄NCl (1) | 73 |
| 16 | Tetramethylharnstoff | Ph₂PPy, MSS (4/4) | 58 |
| 17 | Dimethylpropylenharnstoff | Ph₂PPy, MSS (4/4) | 57 |
| 18 | Dimethylacetamid | Bu₄NCl (1) | 64 |
| NMP = N-Methylpyrrolidon | | | |
| Ph₂PPy = 2-Diphenylphosphinopyridin | | | |
| MSS = Methansulfonsäure | | | |

### Beispiel 19

Eine Mischung aus 26,5 mmol 3-Methoxy-1-buten, 21 mmol trans-1-Methoxy-2-buten, 2,5 mmol cis-1-Methoxy-2-buten, 2,5 mmol Pd(OAc)₂, 5 mmol 1,4-Bis-(diphenylphosphino)-butan und 50 g Toluol wurde in einem 300 ml Autoklaven bei Raumtemperatur mit 10 MPa Kohlenstoffmonoxid versetzt. Anschließend wurde auf 110°C aufgeheizt und 20 h bei dieser Temperatur und dem sich eingestellten Eigendruck (11 MPa) gerührt. Danach wurde auf Raumtemperatur abgekühlt und der Druck auf Atmosphärendruck gebracht. Die Ausbeute an 3-Pentensäuremethylester betrug 35 %.

### Beispiel 20

In einem Autoklav (Volumen 94 ml) mit Magnetrührer, der in einem Ölbad auf 100°C thermostatisiert war, wurden kontinuierlich 5,25 g/h einer Lösung der Zusammensetzung 48,0 Gew.-% Methoxybuten-Isomerengemisch (Molverhältnis 3-Methoxy-1-buten : trans-1-Methoxy-2-buten : cis-1-Methoxy-2-buten : 49:45:6) in N-Methyl-2-pyrrolidon (NMP) und 11,9 g/h Katalysatorlösung der Zusammensetzung 2,20 Gew.-% PdCl₂ und 7,36 Gew.-% Bu₄NCl-Hydrat in NMP sowie 6 l/h gasförmiges CO zugeführt. Der Druck wurde bei 100 bar konstant gehalten. Über ein Regelventil wurden kontinuierlich 21,0 g/h flüssiger Austrag entnommen. Die Ausbeute an 3-Pentensäuremethylester betrug 73,6 % bei einem Umsatz von 85,5 %. Es bildete sich 2-Pentensäuremethylester in einer Ausbeute von 5,1 %. Im Autoklaven war keine Pd-Abscheidung sichtbar. Im flüssigen Reaktionsaustrag konnten 99 Gew.-% des eingesetzten Palladiums in gelöster Form nachgewiesen werden.

### Beispiel 21

Der Versuch aus Beispiel 19 wurde mit dem Unterschied wiederholt, daß 9,10 g/h einer Lösung der Zusammensetzung 48,8 Gew.-% Methoxybuten-Isomerengemisch (Molverhältnis 3-Methoxy-1-buten : trans- 1-Methoxy-2-buten : cis-1-Methoxy-2-buten = 45:50:5) in NMP und 20,6 g/h Katalysatorlösung der Zusammensetzung 2,20 Gew.-% PdCl₂ und 7,36 Gew.-% Bu₄NCl-Hydrat in NMP eingesetzt wurden. Die Ausbeute an 3-Pentensäuremethylester beträgt 69,4 % bei einem Umsatz von 78,9 %. Es bildete sich 2-Pentensäuremethylester in einer Ausbeute von 3,4 %. Im Autoklaven war keine Pd-Abscheidung sichtbar. Im flüssigen Reaktionsaustrag konnte das eingesetzte Palladium quantitativ in gelöster Form wiedergefunden werden.

Das Produkt und nicht umgesetztes Edukt wurden durch Sambaydestillation (100°C, 30 mbar) abgetrennt und der den Katalysator enthaltende Destillationsrückstand anstelle einer frischen Katalysatorlösung erneut eingesetzt. Nach dreimaliger Recyclisierung der Katalysatorlösung in der eben beschriebenen Art wurde eine Ausbeute an 3-Pentensäuremethylester von 69,0 % und eine Ausbeute an 2-Pentensäuremethylester von 3,4 % bei einem Umsatz von 77,9 % erzielt.

## Patentansprüche

1. Verfahren zur Herstellung von 3-Pentensäureestern durch Carbonylierung von Alkoxybutenen in Gegenwart eines Katalysators und eines Lösungsmittels bei erhöhter Temperatur und erhöhtem Druck, dadurch gekennzeichnet, daß man mindestens ein C₁-C₁₀-Alkoxybuten, in dem die Alkoxygruppe in Allylstellung zur Doppelbindung steht, mit Kohlenstoffmonoxid bei einer Temperatur im Bereich von 60 bis 140°C und einem Kohlenmonoxid-Partialdruck im Bereich von 3 bis 30 MPa in Gegenwart eines Katalysators auf der Basis von Palladium in Abwesenheit Halogenid-haltiger Zusatzstoffe umsetzt.

2. Verfahren zur Herstellung von 3-Pentensäureestern durch Carbonylierung von Alkoxybutenen in Gegenwart eines Katalysators und eines Lösungsmittels bei erhöhter Temperatur und erhöhtem Druck, dadurch gekennzeichnet, daß man mindestens ein C₁-C₁₀-Alkoxybuten, in dem die Alkoxygruppe in Allylstellung zur Doppelbindung steht, mit Kohlenstoffmonoxid bei einer Temperatur im Bereich von 60 bis 140°C und einem Kohlenmonoxid-Partialdruck im Bereich von 3 bis 30 MPa in Gegenwart eines Katalysators auf der Basis von Palladium und eines Zusatzstoffes ausgewählt aus der Gruppe bestehend aus Chloriden, Säuren, Stickstoff-haltige Liganden und Phosphorhaltige Liganden bei einem molaren Verhältnis von Zusatzstoff zu Katalysator von 0,1 bis 10 umsetzt.

3. Verfahren nach Anspruch 1 oder 2, dadurch gekennzeichnet, daß man eine Mischung aus trans-1-Methoxy-2-buten und cis-1-Methoxy-2-buten einsetzt.

4. Verfahren nach Anspruch 1 oder 2, dadurch gekennzeichnet, daß man eine Mischung aus 3-Methoxy-1-buten, trans-1-Methoxy-2-buten und cis-1-Methoxy-2-buten einsetzt.

## Claims

1. A process for preparing 3-pentenoic esters by carbonylation of alkoxybutenes in the presence of a catalyst and a solvent at elevated temperature and elevated pressure, which comprises reacting at least one C₁-C₁₀-alkoxybutene in which the alkoxy group is in the allyl position relative to the double bond with carbon monoxide at from 60 to 140°C and a carbon monoxide partial pressure in the range from 3 to 30 MPa in the presence of a catalyst based on palladium in the absence of halide-containing additives.

2. A process for preparing 3-pentenoic esters by carbonylation of alkoxybutenes in the presence of a catalyst and a solvent at elevated temperature and elevated pressure, which comprises reacting at least one C₁-C₁₀-alkoxybutene in which the alkoxy group is in the allyl position relative to the double bond with carbon monoxide at from 60 to 140°C and a carbon monoxide partial pressure in the range from 3 to 30 MPa in the presence of a catalyst based on palladium and an additive selected from the group consisting of chlorides, acids, nitrogen-containing ligands and phosphorus-containing ligands at a molar ratio of additive to catalyst of from 0.1 to 10.

3. A process as claimed in claim 1 or 2, wherein a mixture of trans-1-methoxy-2-butene and cis-1-methoxy-2-butene is used.

4. A process as claimed in claim 1 or 2, wherein a mixture of 3-methoxy-1-butene, trans-1-methoxy-2-butene and cis-1-methoxy-2-butene is used.

## Revendications

1. Procédé de préparation d'esters d'acide 3-penténique par carbonylation d'alcoxybutènes en présence d'un catalyseur et d'un solvant à température élevée et pression élevée, caractérisé en ce qu'on fait réagir au moins un C₁-C₁₀-alcoxybutène, dans lequel le groupe alcoxy est en position allyle par rapport à la double liaison, avec du monoxyde de carbone à une température de l'ordre de 60 à 140°C et à une pression partielle de monoxyde de carbone de l'ordre de 3 à 30 MPa, en présence d'un catalyseur à base de palladium et en l'absence d'additifs contenant un halogénure.

2. Procédé de préparation d'esters d'acide 3-penténique par carbonylation d'alcoxybutènes en présence d'un catalyseur et d'un solvant à température élevée et pression élevée, caractérisé en ce qu'on fait réagir au moins un C₁-C₁₀-alcoxybutène, dans lequel le groupe alcoxy est en position allyle par rapport à la double liaison, avec du monoxyde de carbone à une température de l'ordre de 60 à 140°C et à une pression partielle de monoxyde de carbone de l'ordre de 3 à 30 MPa, en présence d'un catalyseur à base de palladium et d'un additif choisi parmi le groupe constitué de chlorures, d'acides, de ligands contenant de l'azote et de ligands contenant du phosphore, à un rapport molaire entre l'additif et le catalyseur de 0,1 à 10.

3. Procédé suivant l'une des revendications 1 et 2, caractérisé en ce qu'on met en oeuvre un mélange de trans-1-méthoxy-2-butène et de cis-1-méthoxy-2-butène.

4. Procédé suivant l'une des revendications 1 et 2, caractérisé en ce qu'on met en oeuvre un mélange de 3-méthoxy-1-butène, de trans-1-méthoxy-2-butène et de cis-1-méthoxy-2-butène.
